# DEMANDE DE BREVET EUROPEEN

(11) **EP 1 550 437 A2**
(43) Date de publication de la demande: **06.07.2005**
(21) Numéro de dépôt: 04292886.1
(22) Date de dépôt: 06.12.2004
(51) Int. Cl.: A61K 7/48, A61K 47/32

(54) **Composition comprenant un polymère semi-cristallin et un copolymère de vinylpyrrolidone/alfa-oléfine**

(30) Priorité: 05.01.2004 JP 2004000712
(71) Demandeur: L'OREAL, 75008 Paris (FR)
(72) Inventeur: Inoue, Mika, 206-0002 Tokyo (JP); Kawashima, Noriko, Yokohama-shi 224-0055 Kanagawa-ken (JP); Okamoto, Mariko, 113-0021 Tokyo (JP)
(74) Mandataire: Rasson, Catherine

(57) **Abrégé**

Problème : Offrir une composition sous forme de pâte ou de crème destinée à une application externe présentant une excellente facilité d'utilisation, une stabilité élevée et une bonne applicabilité à la peau et analogues, ainsi qu'un procédé de préparation d'une telle composition en vue d'une application externe. Solution : Une composition non solide sous forme de pâte ou de crème destinée à une application externe est préparée en mélangeant au moins un polymère semi-cristallin ayant un point de fusion d'au moins 50°C, et au moins une copolymère de polyvinylpyrrolidone/α-oléfine ayant un point de fusion d'au moins 50°C, avec une phase huileuse comprenant au moins une huile non volatile, en gélifiant la phase huileuse, et après la gélification ou pendant la gélification de la phase huileuse, en appliquant une force de cisaillement prédéterminée pour fragmenter la structure gélifiée.

## Description

### Domaine technique de l'invention

L'invention concerne en général des compositions destinées à une application externe telle que des produits cosmétiques et des produits analogues, et elle concerne plus spécifiquement des compositions sous forme de pâte ou de crème destinées à une application externe présentant une excellente facilité d'utilisation, une stabilité élevée et une bonne applicabilité à la peau et analogues. De plus, la présente invention concerne également un procédé de préparation d'une telle composition en vue d'une application externe.

### Art Conventionnel

Les compositions cosmétiques à base d'huile peuvent être en grande partie divisées en des compositions cosmétiques liquides à base d'huile et des compositions cosmétiques solides à base d'huile.

Par exemple, tandis que les produits démaquillants qui sont actuellement commercialisés comprennent généralement des produits sous forme de crème, d'émulsion, d'huile et de gel, les démaquillants à base d'huile (nommés huiles nettoyantes) sont très appréciés des femmes pour avoir un effet démaquillant extrêmement élevé, car ils pénètrent facilement dans la peau, et ils ne laissent pas de traces huileuses sur la peau après rinçage avec de l'eau en raison de l'inclusion de tensioactifs.

De plus, les fonds de teint d'eau dans l'huile ou non aqueux sous forme de crème, d'émulsion et de liquide sont généralement connus. Les fonds de teint liquides comprennent ceux sous forme biphasique, qui doivent être agités afin d'obtenir une bonne homogénéisation juste avant l'utilisation. Les fonds de teint liquides ou biphasiques sont également très appréciés car ils sont faciles à étaler, forment un film maquillant mince, et laissent une sensation légère. En outre, les émulsions d'écran total sous forme de liquides biphasique d'eau dans l'huile sont également largement répandues, et sont utilisées en agitant afin d'obtenir une bonne homogénéisation juste avant l'utilisation.

Cependant, étant donné que ces produits sous forme liquide et semi-liquide présentent une fluidité élevée, ils peuvent éclabousser lorsqu'on les verse sur les mains, ou couler lorsqu'on les applique avec les mains sur le visage, risquant ainsi de salir les vêtements. De plus, il est beaucoup plus difficile d'enlever des tâches sur des vêtements de ce type de compositions cosmétiques d'eau dans l'huile et de produits non aqueux à base d'huile, que de produits cosmétiques à base d'eau.

D'autre part, les compositions cosmétiques solides sont courantes dans les produits de soin pour la peau et les produits de maquillage, et se présentent souvent sous forme compacte ou de bâton. Ces types de produits cosmétiques sont commodes à transporter et à porter, sans risque d'être déversés.

La plupart de ces compositions cosmétiques solides sont des gels non aqueux qui ne contiennent pas d'eau, et de tels gels, particulièrement lorsqu'ils sont sous forme de bâtons, ont l'inconvénient d'être difficiles à étaler lorsqu'ils sont appliqués sur la peau à cause de leur propriété de dureté. De plus, lorsqu'ils sont sous une forme compacte fondue, une éponge est nécessaire pour les appliquer sur la peau. En outre, avec ces produits, le bâton et la peau ou l'éponge et la peau sont en contact direct lors de l'application sur la peau, ce qui n'est pas souhaitable en termes d'hygiène.

Ainsi, les inventeurs ont essayés de surmonter les inconvénients engendrés par les compositions cosmétiques liquides à base d'huile en créant une phase huileuse dans une composition cosmétique à base d'huile à l'aide d'un épaississant ou analogue, mais ils n'ont pas pu obtenir de résultats satisfaisants.

### Brevet 1

### JP-A 2003-40739

### Brevet 2

### JP-A H7-89826

### Problèmes devant être résolus par l'invention

Par conséquent, l'objet principal de la présente invention est de proposer une composition destinée à une application externe telle qu'une composition cosmétique non solide qui surmonte les problèmes rencontrés dans l'art conventionnel décrits ci-dessus, et un procédé de préparation de celle-ci.

Un autre objet de la présente invention est de proposer une composition destinée à une application externe sous forme d'une pâte ou d'une crème présentant une excellente stabilité, en particulier une stabilité aux températures élevées, et un procédé de préparation de celle-ci.

Un autre objet de la présente invention est de proposer une composition destinée à une application externe parmi les compositions cosmétiques à base d'huile contenant un système émulsionné, fournissant des effets de sorte qu'il soit facile de prendre sur un doigt une quantité appropriée, qu'elle puisse être aisément étalée lorsqu'elle est appliquée sur la peau, et qu'elle soit légère au toucher, du au fait qu'elle soit sous forme d'une pâte ou d'une crème, et un procédé de préparation de celle-ci.

### Moyens de résoudre les problèmes

Les présents inventeurs ont découvert, à leur surprise, qu'en utilisant deux types spéciaux de polymères pour gélifier une phase huileuse non volatile, et en détruisant la structure gélifiée de celle-ci, ou en empêchant la progression de la gélification, il est possible d'obtenir une composition cosmétique présentant une excellente stabilité, et ayant également au moins certaines des diverses propriétés souhaitables décrites ci-dessus.

Ainsi, la présente invention propose une composition destinée à une application externe comprenant :
(A) au moins un polymère semi-cristallin ayant un point de fusion d'au moins 50°C, de préférence compris entre 50 et 120°C, plus préférablement compris entre 50 et 70°C ;
(B) au moins un copolymère de polyvinylpyrrolidone/α-oléfine ayant un point de fusion d'au moins 50°C, de préférence compris entre 50 et 120°C, plus préférablement compris entre 50 et 70° C ; et
(C) une phase huileuse contenant au moins une huile non volatile ; et prenant la forme d'une pâte ou d'une crème à température ambiante.

La composition destinée à une application externe selon la présente invention a une viscosité optimale pour présenter une excellente facilité d'utilisation, présente une stabilité élevée, particulièrement une stabilité aux températures élevées, il est particulièrement facile de prendre une quantité appropriée sur un doigt, elle s'étale bien lorsqu'elle est appliquée sur la peau, ou pénètre aisément dans la peau, et laisse un toucher léger.

Le fait que les phases huileuses liquides puissent être structurées en utilisant des polymères semi-cristallins pour obtenir des compositions cosmétiques telles que des bâtons de rouge à lèvre est déjà connu. Par exemple, le brevet 1 décrit une composition comprenant essentiellement un polymère semi-cristallin avec un faible point de fusion inférieur à 50°C et comprenant éventuellement un polymère semi-cristallin ayant un point de fusion élevé d'au moins 50°C. Cependant, afin d'obtenir une composition sous forme de pâte ou de crème, le fait de réduire simplement la quantité de polymère semi-cristallin n'a pas conduit à une stabilité, particulièrement une stabilité à une température élevée de 45°C ou plus.

De plus, l'utilisation d'un copolymère de vinylpyrrolidone pour structurer une phase huileuse liquide afin d'obtenir une composition cosmétique sous forme de bâton est également connue (par exemple, voir le brevet 2). Par conséquent, des tentatives ont été faites pour obtenir un produit sous forme de pâte ou de crème en réduisant simplement la quantité de copolymère de vinylpyrrolidone ; mais dans ce cas aussi, le produit présentait une stabilité faible, particulièrement une stabilité à une température élevée de 45°C ou plus.

Par conséquent, la composition de la présente invention est préparée à l'aide d'un procédé de préparation d'une composition destinée à une application externe, qui consiste à mélanger au moins un polymère semi-cristallin ayant un point de fusion d'au moins 50°C, et au moins un copolymère de polyvinylpyrrolidone/α-oléfine ayant un point de fusion d'au moins 50°C, avec une phase huileuse comprenant au moins une huile non volatile ; à gélifier ladite phase huileuse ; et à appliquer une force de cisaillement après la gélification ou pendant la gélification pour obtenir une structure gélifiée fragmentée.

Dans la présente invention, la structure gélifiée peut être fragmentée par n'importe quel procédé, et elle peut prendre diverses formes selon la capacité de production, la façon dont sera utilisée la composition donnée, les types et les quantités des ingrédients éventuels contenus dans la composition, et les types d'appareils qui peuvent être utilisés.

Par exemple, dans un mode de réalisation, la composition selon la présente invention est préparée en mélangeant un polymère semi-cristallin et un copolymère de polyvinylpyrrolidone/α-oléfine avec une phase huileuse, ensuite en gélifiant la phase huileuse pour préparer une composition gélifiée solide. Ensuite, la composition résultante est malaxée à l'aide d'un malaxeur tel que, par exemple, un broyeur à trois cylindres, pour obtenir une structure gélifiée fragmentée.

De plus, dans un autre mode de réalisation, la composition selon la présente invention peut être préparée en mélangeant un polymère semi-cristallin et un copolymère de polyvinylpyrrolidone/α-oléfine avec une phase huileuse, en chauffant afin d'obtenir la dissolution, puis en utilisant un appareil d'agitation quelconque pour refroidir tout en agitant, pour obtenir un structure gélifiée fragmentée.

Dans un autre mode de réalisation, la composition selon la présente invention peut être préparée en mélangeant un polymère semi-cristallin et un copolymère de polyvinylpyrrolidone/α-oléfine avec une phase huileuse, en chauffant afin d'obtenir la dissolution, puis en passant la composition résultante dans une extrudeuse, pour obtenir une structure gélifiée fragmentée.

### Modes de réalisation de l'invention

Ci-après, les modes de réalisation de la présente invention sont décrits en détail.

La composition destinée à une application externe obtenue par la présente invention est une composition qui forme une pâte ou une crème à température ambiante.

Dans la présente invention, l'expression "forme de pâte ou de crème" désigne une forme non solide, non liquide, c.-à-d. un produit mou, comme l'est généralement compris par l'homme du métier, et comprend également les formes fluides qui sont souvent exprimées par d'autres termes, telles que les gels. De plus, les compositions sous forme de pâte ou de crème peuvent être soit à base d'huile soit à base d'eau, et peuvent prendre la forme de systèmes émulsionnés tels que des systèmes émulsionnés d'huile dans l'eau, des systèmes émulsionnés d'eau dans l'huile, et des gels ou des pâtes non aqueux, les systèmes émulsionnés non aqueux ou d'eau dans l'huile étant préférables.

Dans une forme de la présente invention, la propriété d'être "sous forme de pâte ou de crème" selon la composition de la présente invention peut être exprimée par des propriétés physico-chimiques mesurables spécifiques. Par exemple, la propriété d'être une pâte ou une crème peut être exprimée par une gamme spécifique de viscosité, et par exemple, elle peut être définie de sorte qu'elle ait une viscosité mesurable sur les rotors numéros 2, 3, 4 et 5 pendant 10 minutes à un taux de cisaillement de 200 s⁻¹ en utilisant un Rheomat RM180 (Rheometric Scientific), de préférence elle est définie comme ayant une viscosité comprise entre 330 centipoises (limite inférieure, lecture de 50 sur le rotor numéro 2) et 35 000 centipoises (limite supérieure, lecture de 30 sur le rotor numéro 5), et plus préférablement entre 650 centipoises (limite inférieure, lecture de 20 sur le rotor numéro 3) et 11 800 centipoises (limite supérieure, lecture de 50 sur le rotor numéro 4) à température ambiante.

La composition sous forme de pâte ou de crème de la présente invention est caractérisée en ce qu'elle comprend au moins un type de polymère semi-cristallin ayant un point de fusion d'au moins 50°C, au moins un type de copolymère de polyvinylpyrrolidone/α-oléfine ayant un point de fusion d'au moins 50°C, et une phase huileuse contenant au moins un type d'huile non volatile. Ci-après, les ingrédients nécessaires pour la composition selon la présente invention sont décrits.

### A. Polymères semi-cristallins

Dans le cadre de la présente invention, le terme "polymère semi-cristallin" a la même signification que celle qui est comprise par l'homme du métier, en particulier les polymères comprenant une chaîne principale polymérique organique et une chaîne latérale (chaîne pendante), comprenant une partie cristallisable dans une partie de la chaîne latérale et/ou de la chaîne principale, et une partie amorphe dans la chaîne principale et présentant une température de transition de phase du premier ordre réversible, telle qu'une température de fusion (transition solide-liquide). Le terme "polymères" est compris comme signifiant, selon la présente invention, des composés comprenant au moins deux unités de répétition, telles qu'au moins 3 unités de répétition et aussi telles qu'au moins 10 unités de répétition. Si la partie cristalline est une séquence de chaîne principale polymérique, alors les propriétés chimiques de cette séquence cristalline différeront de celles d'une séquence amorphe. Dans ce cas, un polymère semi-cristallin est un polymère à blocs, et peut, par exemple, être de type diblocs, triblocs ou multiblocs. Le polymère semi-cristallin utilisé dans cette invention est soluble ou dispersable dans la phase huileuse.

Le polymère ou les polymères semi-crystallins de la composition de l'invention comprennent un poids moléculaire moyen en nombre Mn avantageux d'au moins 2000, allant de 2000 à 800 000, préférablement de 3000 à 500 000, par exemple, de 4000 à 150 000. Plus préférablement, le poids moléculaire moyen en nombre est inférieur à 100 000, en particulier de 4000 à 99 000. En outre, ils peuvent présenter un poids moléculaire moyen en nombre supérieur à 5600, par exemple, allant de 5700 à 99 000.

Selon l'invention, les polymères semi-cristallins sont solubles dans la phase huileuse à au moins 1 % en poids à une température supérieure à leur température de fusion. Indépendamment des chaînes ou des blocs cristallisables, les séquences de polymère sont amorphes. Le terme "chaîne ou bloc cristallisable" est compris comme signifiant, selon la présente invention, une chaîne ou un bloc qui, si elle était seule, changerait réversiblement de l'état amorphe à l'état cristallin, selon que la température est supérieure ou inférieure à la température de fusion. Une chaîne au sens de l'invention est un groupe d'atomes qui est en position pendante ou latérale par rapport à la chaîne principale polymérique. Un bloc est un groupe d'atomes appartenant à la chaîne principale, un groupe constituant une des unités de répétition du polymère. De préférence, la chaîne principale polymérique des polymères semi-cristallins est soluble dans la phase huileuse.

De plus, le polymère semi-cristallin de la présente invention a un point de fusion élevé Pf, de préférence tel que 50°C ≤ Pf ≤ 120°C, plus préférablement tel que 50°C ≤ Pf ≤ 100°C, et aussi tel que 50°C ≤ Pf ≤ 70°C. Ce point de fusion est une température de transition d'état du premier ordre. Selon l'invention, le point de fusion peut être mesuré par n'importe quel procédé connu, tel qu'à l'aide d'un calorimètre à balayage différentiel (DSC). Si le point de fusion est inférieur à 50°C, la force de solidification est insuffisante, ce qui est indésirable en raison de la stabilité à température élevée du produit. De plus, si le point de fusion est supérieur à 120°C, un traitement à température élevée devient nécessaire afin d'obtenir la dissolution, ce qui n'est pas désirable.

Par exemple, le bloc ou la chaîne ou les blocs ou les chaînes cristallisables des polymères semi-cristallins représentent au moins 30 % du poids total de chaque polymère tel qu'au moins 40 %. Les polymères semi-cristallins de l'invention avec des blocs cristallisables sont des polymères à blocs ou multiblocs. Ils peuvent être obtenus par polymérisation d'un monomère avec des doubles liaisons réactives (ou éthyléniques) ou par polycondensation. Lorsque les polymères semi-cristallins de l'invention sont des polymères ayant des chaînes latérales cristallisables, ces dernières sont de préférence sous forme aléatoire.

Les polymères semi-cristallins de l'invention peuvent être d'origine synthétique, et peuvent ne pas comprendre une chaîne principale de type polysaccharide.

Selon l'invention, le polymère semi-cristallin est choisi parmi les copolymères à blocs comprenant au moins un bloc cristallisable et au moins un bloc amorphe, les homopolymères et les copolymères portant au moins une chaîne latérale cristallisable par unité de répétition, et les mélanges de ceux-ci.

Les polymères semi-cristallins qui peuvent être utilisés dans l'invention sont :
(1) les copolymères à blocs de polyoléfines avec une cristallisation contrôlée, tels que ceux pour lesquels les monomères sont décrits dans le brevet EP-A-0 951 897 ;
(2) les polycondensats de type polyester aliphatique ou aromatique ou de type copolyester aliphatique/aromatique ;
(3) les homo- et copolymères portant au moins une chaîne latérale cristallisable et les homo- ou copolymères portant, dans la chaîne principale, au moins un bloc cristallisable, tel que ceux décrits dans le brevet US 5 156 911 ;
(4) les homo- et copolymères portant au moins une chaîne latérale cristallisable avec un ou des groupes fluorés, tel que ceux décrits dans le document WO-A-01/19333 ; et
(5) les mélanges de ceux-ci.

Dans les deux derniers cas (3 et 4), la chaîne latérale ou le bloc ou les chaînes latérales ou les blocs cristallisables sont hydrophobes.

### a) Polymères semi-cristallins avec des chaînes latérales cristallisables

On peut mentionner ceux définis dans les brevets US 5 156 911 et WO-A-01/19333.

Ce sont des homopolymères ou des copolymères comprenant de 50 à 100 % en poids d'unités, résultant de la polymérisation d'un ou plusieurs monomères portant une chaîne latérale hydrophobe cristallisable.

Ces homo- ou copolymères peuvent avoir une nature quelconque à condition qu'ils présentent les conditions indiquées ci-dessous, avec par exemple, la caractéristique d'être solubles ou dispersables dans la phase huileuse en chauffant à une température supérieure à leur température de fusion Pf. Ils peuvent résulter :
- de la polymérisation, telle que la polymérisation de radicaux libres, d'un ou plusieurs monomères avec une ou plusieurs doubles liaisons ou de monomères éthyléniques réactifs vis-à-vis de la polymérisation, à savoir avec un groupe vinyle, (méth)acrylique ou allyle ; et
- de la polycondensation d'un ou plusieurs monomères portant des groupes coréactifs (des groupes acide carboxylique ou sulfonique, alcool, amine ou isocyanate), tels que par exemple, des polyesters, des polyuréthanes, des potyéthers, des polyurées ou des polyamides.

Généralement, ces polymères peuvent être choisis parmi les homopolymères et les copolymères résultant de la polymérisation d'au moins un monomère avec une ou des chaînes cristallisables, qui peuvent être représentés par la formule (I) : 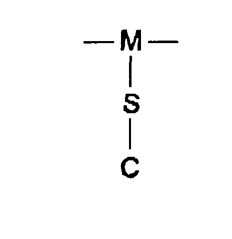 M représentant un atome de la chaîne principale polymérique, S représentant un espaceur, et C représentant un groupe cristallisable.

Les chaînes cristallisables "-S-C" peuvent être aliphatiques ou aromatiques et éventuellement fluorées ou perfluorées. "S" peut représenter un groupe linéaire ou ramifié ou cyclique (CH₂)ₙ ou (CH₂CH₂O)ₙ ou (CH₂O), n étant un nombre entier allant de 0 à 22. Par exemple, "S" est un groupe linéaire. Selon un autre aspect de l'invention, "S" et "C " sont différents.

Lorsque les chaînes cristallisables "-S-C" sont des chaînes aliphatiques hydrocarbonées, elles comprennent des chaînes alkyle hydrocarbonées avec au moins 11 atomes de carbone et au plus 40 atomes de carbone, tels que au plus 24 atomes de carbone. Elles peuvent être des chaînes aliphatiques ou des chaînes alkyle ayant au moins 12 atomes de carbone ; selon un aspect de l'invention, elles sont des chaînes alkyle en C₁₄-C₂₄. Lorsque ce sont des chaînes alkyle fluorées ou perfluorées, elles comprennent au moins 6 atomes de carbone fluorés et peuvent avoir au moins 11 atomes de carbone, dont au moins 6 atomes de carbone sont fluorés.

On peut mentionner, en tant qu'exemples non limitatifs de polymères semi-cristallins avec une ou des chaînes cristallisables, ceux qui résultent de la polymérisation d'un ou plusieurs monomères suivants : les (méth)acrylates d'alkyle saturé avec un groupe alkyle en C₁₄-C₂₄ ; les (méth)acrylates de perfluoroalkyle avec un groupe perfluoroalkyle en C₁₁-C₁₅ ; les (méth)acrylamides de N-alkyle avec un groupe alkyle en C₁₄ à C₂₄, avec ou sans atome de fluor ; les esters vinyliques avec des chaînes alkyle ou perfluoro(alkyle) avec un groupe alkyle en C₁₄ à C₂₄ (avec au moins 6 atomes de fluor par chaîne perfluoroalkyle) ; les éthers de vinyle avec des chaînes alkyle ou perfluoro(alkyle) avec le groupe alkyle en C₁₄ à C₂₄ et au moins 6 atomes de fluor par chaîne perfluoroalkyle ; les α-oléfines en C₁₄ à C₂₄ telles que, par exemple, l'octadécène ; les para-alkylstyrènes avec un groupe alkyle comprenant de 12 à 24 atomes de carbone, et les mélanges de ceux-ci.

Le terme "alkyle" est compris comme signifiant, selon la présente invention, un groupe saturé, par exemple, un groupe en C₈-C₂₄, sauf indication contraire, et également un groupe en C₁₄-C₂₄.

Lorsque les polymères résultent d'une polycondensation, les chaînes hydrocarbonées et/ou fluorées cristallisables telles que définies ci-dessus sont portées par un monomère qui peut être un diacide, un diol, une diamine ou un diisocyanate.

Lorsque les polymères qui constituent l'objet de l'invention sont des copolymères, ils comprennent d'autre part de 0 à 50 % de groupes de type Y ou Z résultant de la copolymérisation :
α) de Y, qui est un monomère polaire ou non polaire ou un mélange des deux :
   - lorsque Y est un monomère polaire, il s'agit d'un monomère portant des groupes polyoxyalkylés (par exemple, des groupes oxyéthylés et/ou oxypropylés) ; un (méth)acrylate d'hydroxyalkyle, tel que l'acrylate d'hydroxyéthyle ; le (méth)acrylamide ; un (méth)acrylamide de N-alkyle ; un (méth)acrylamide de N,N-dialkyle, tel que, par exemple, le N,N-diisopropylacrylamide ; la N-vinylpyrrolidone (NVP) ; le N-vinylcaprolactame ; ou un monomère portant au moins un groupe acide carboxylique, tel que l'acide (méth)acrylique, l'acide crotonique, l'acide itaconique, l'acide maléique ou l'acide fumarique, ou portant un groupe anhydride d'acide carboxylique, tel que l'anhydride maléique, et les mélanges de ceux-ci ;
   - lorsque Y est un monomère non polaire, il peut être un ester de type (méth)acrylate d'alkyle linéaire, ramifié ou cyclique, un ester vinylique, un éther de vinyle et d'alkyle, une α-oléfine, le styrène ou un styrène substitué par un groupe alkyle en C₁-C₁₀, tel que l'α-méthylstyrène, ou un macromonomère de type polyorganosiloxane avec une insaturation vinylique ;
β) de Z, qui est un monomère polaire ou un mélange de monomères polaires. Dans ce cas, Z a la même définition que le monomère "Y polaire" défini ci-dessus.

Par exemple, les polymères semi-crystallins avec une chaîne latérale cristallisable sont des homopolymères de (méth)acrylate d'alkyle ou de (méth)acrylamide d'alkyle avec un groupe alkyle comme défini ci-dessus tel qu'un groupe alkyle en C₁₄-C₂₄, les copolymères de ces monomères avec un monomère hydrophile peuvent être de nature différente de l'acide (méth)acrylique, tel que la N-vinylpyrrolidone ou le (méth)acrylate d'hydroxyéthyle, et les mélanges de ceux-ci.

### b) Polymères portant, dans la chaîne principale, au moins un bloc cristallisable

Ce sont à nouveau des polymères qui sont solubles ou dispersables dans la phase huileuse en chauffant à une température supérieure à leur point de fusion Pf. Ces polymères peuvent être des copolymères à blocs composés d'au moins deux blocs de natures chimiques différentes, dont l'un d'entre eux est cristallisable.

Les types suivants de polymères peuvent être utilisés :
- Les polymères définis dans le brevet US 5 156 911.
- Les copolymères à blocs à base d'oléfine ou de cyclooléfine avec une chaîne cristallisable, tels que ceux résultant de la polymérisation des blocs de type :
   - cyclobutène, cyclohexène, cyclooctène, norbornène (c'est-à-dire, le bicyclo[2,2,1]hept-2-ène), 5-méthylnorbornène, 5-éthylnorbornène, 5,6-diméthylnorbornène, 5,5,6-triméthylnorbornène, 5-éthylidènenorbornène, 5-phénylnorbornène, 5-benzylnorbornène, 5-vinylnorbornène, 1,4,5,8-diméthano-1,2,3,4,4a,5,8a-octahydronaphthalène, dicyclopentadiène ou des mélanges de ceux-ci, avec
   - éthylène, propylène, 1-butène, 3-méthyl-1-butène, 1-hexène, 4-méthyl-1-pentène, 1-octène, 1-decène, 1-eicosène ou des mélanges de ceux-ci.

Ces polymères à blocs peuvent être par exemple, des blocs copoly(éthylène/norbornène) et des blocs de terpolymère (éthylène/propylène/éthylidènenorbornène). On peut également utiliser ceux qui résultent de la copolymérisation en blocs d'au moins 2 α-oléfines en C₂-C₁₆ par exemple, les α-oléfines en C₂-C₁₂, telles que celles mentionnées ci-dessus, et aussi telles que les bipolymères à blocs d'éthylène et de 1-octène.
- Les copolymères présentant au moins un bloc cristallisable, le reste du copolymère étant amorphe (à température ambiante).

Ces copolymères peuvent, de plus, présenter deux blocs cristallisables de natures chimiques différentes. Les exemples de copolymères sont ceux qui ont, à température ambiante, à la fois un bloc cristallisable et un bloc amorphe hydrophobe et lipophile qui sont distribués de façon séquentielle ; on peut mentionner, par exemple, les polymères ayant l'un des blocs cristallisables suivants et l'un des blocs amorphes suivants :
- bloc cristallisable de nature : a) polyester, tel que les poly(alkylène téréphthalate)s, b) polyoléfine, telle que les polyéthylènes ou les polypropylènes.
- bloc amorphe et lipophile, tel que : les polyoléfines ou copoly(oléfine)s amorphes, par exemple, le poly(isobutylène), le polybutadiène hydrogéné ou le poly(isoprène) hydrogéné.
   On peut mentionner, en tant qu'exemples non limitatifs appropriés de tels copolymères avec un bloc cristallisable et avec un bloc amorphe :
   α) les copolymères à blocs de poly(ε-caprolactone)-b-poly(butadiène), tels que ceux utilisés sous forme hydrogénée, tels que ceux décrits dans l'article, "Melting Behavior of poly(ε-caprolactone)-block-polybutadiene copolymers", de S. Nojima, Macromolecules, 32, 3727-3734 (1999) ;
   β) les copolymères à un bloc ou multiblocs de poly(butylène téréphtalate)-b-poly(isoprène) hydrogéné, cités dans l'article, "Study of Morphological and Mechanical Properties of PP/PBT", de B. Boutevin et al., Polymer Bulletin, 34, 117-123 (1995) ; y) les copolymères à blocs de poly(éthylène)-b-copoly(éthylène/propylène) cités dans les articles, "Morphology of Semi-Crystalline Block Copolymers of ethylene-(ethylene-alt-propylene)" de P. Rangarajan et al., Macromolecules, 26, 4640-4645 (1993) et "Polymer Aggregates with Crystalline Cores: the System poly(ethylene)-poly(ethylene-propylene)", P. Richter et al., Macromolecules, 30, 1053-1068 (1997) ; et
   δ) les copolymères à blocs de poly(éthylène)-b-poly(éthyléthylène) cités dans l'article général, "Crystallization in Block Copolymers", de I.W. Hamley, Advances in Polymer Science, vol. 148, 113-137 (1999).

Les polymères semi-cristallins de la composition de l'invention peuvent être ou peuvent ne pas être partiellement réticulés à condition que le degré de réticulation ne nuise pas à leur dissolution ou dispersion dans la phase huileuse en chauffant à une température supérieure à leur température de fusion. La réticulation peut ensuite être une réticulation chimique, par réaction avec un monomère multifonctionnel pendant la polymérisation. Elle peut également être une réticulation physique qui peut ensuite être due à l'établissement de liaisons de type hydrogène ou dipolaire entre les groupes portés par le polymère telles que, par exemple, des interactions dipolaires entre des ionomères carboxylates, ces interactions étant faibles en degré et portées par la chaîne principale polymérique, ou bien à une séparation de phase entre les blocs cristallisables et les blocs amorphes portés par le polymère.

Les polymères semi-cristallins de la composition selon l'invention peuvent, par exemple, être non réticulés.

Selon un mode de réalisation de l'invention, le polymère peut être choisi parmi les copolymères résultant de la polymérisation d'au moins un monomère avec une chaîne cristallisable, choisis parmi les (méth)acrylates d'alkyle en C₁₄-C₂₄ saturé ; les (méth)acrylates de perfluoroalkyle en C₁₁-C₁₅ ; les (méth)acrylamides de N-( alkyle en C₁₄-C₂₄), avec ou sans au moins un atome de fluor ; les esters vinyliques avec des chaînes alkyle ou perfluoroalkyle en C₁₄ à C₂₄ ; les éthers de vinyle avec des chaînes alkyle ou perfluoroalkyle en C₁₄ à C₂₄ ; les α-oléfines en C₁₄ à C₂₄ ; ou les para-alkylstyrènes avec un groupe alkyle comprenant de 12 à 24 atomes de carbone, avec au moins un ester ou une amide d'acide monocarboxylique en C₁ à C₁₀ éventuellement fluoré, qui peut être représenté par la formule suivante : 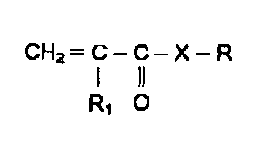 dans laquelle R₁ est choisi parmi H et CH₃, R est choisi parmi les groupes alkyle en C₁-C₁₀ éventuellement fluorés, et X est choisi parmi O, NH et NR₂, où R₂ représente un groupe alkyle en C₁-C₁₀ éventuellement fluoré.

Selon un mode de réalisation de l'invention, le polymère peut résulter d'un monomère avec une chaîne cristallisable choisie parmi les (méth)acrylates d'alkyle en C₁₄-C₂₂ saturé.

On peut mentionner, en tant qu'exemples non limitatifs de polymères semi-cristallins structurants qui peuvent être utilisés dans la composition selon l'invention, les produits Intelimer® de Landec décrits dans la brochure "Intelimer® polymers", Landec IP22 (Rev. 4-97). Ces polymères sont sous forme solide à température ambiante. Ils portent des chaînes latérales cristallisables et présentent la formule (I) mentionnée ci-dessus.

On peut utiliser des polymères semi-cristallins avec un point de fusion d'au moins 50°C obtenus par la copolymérisation de l'acrylate de béhényle et de l'acide acrylique ou de NVP comme décrit dans le brevet US 5 519 063, tels que ceux décrits dans les exemples 1 et 2 de la préparation de polymères ci-dessous, avec des températures de fusion de 60°C et 58°C, respectivement.

Dans un exemple, le polymère semi-cristallin ne comprend pas de groupe carboxylique.

On peut utiliser également des polymères semi-cristallins décrits dans l'application US 2001/0018484.

De préférence, le polymère semi-cristallin utilisé dans la présente application est un homopolymère obtenu par polymérisation de l'acrylate de béhényle, particulièrement le polymère appelé "Intelimer IPA-13.6" de Landec Corporation. Ce polymère a une température de fusion de 66°C.

La composition de la présente invention contient les polymères semi-cristallins en une quantité allant de 0,1 à 14 % en poids, de préférence de 1 à 5 % en poids par rapport au poids total de la composition.

### B. Copolymère de vinylpyrrolidone/α-oléfine

Les copolymères de polyvinylpyrrolidone/α-oléfine utilisés dans la présente invention ont un point de fusion d'au moins 50°C, sont lipophiles, et peuvent être exprimés par la formule suivante : 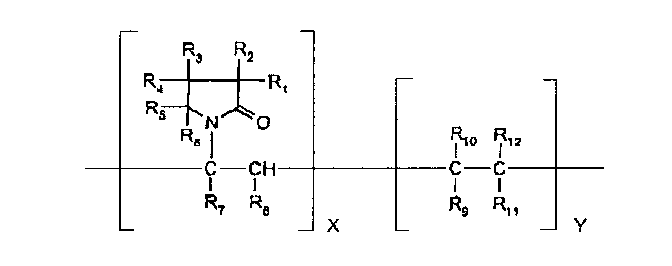 dans laquelle les radicaux R₁ à R₁₂ représentent, indépendamment les uns des autres, une groupe alkyle saturé linéaire ou ramifié en C₁₀ à C₄₀ ou un atome d'hydrogène, au moins un des groupes R₁ à R₁₂ mentionnés ci-dessus étant différent d'un atome d'hydrogène. La valeur de Y peut être égale à 0 ; X ne doit pas être égal à 0.

De préférence, au moins un des radicaux qui ne sont pas des atomes d'hydrogène contient 14 à 32 atomes de carbone, plus préférablement 28 à 32 atomes de carbone.

Parmi les groupes alkyle ayant 10 à 40 atomes de carbone, il est possible de citer en tant qu'exemples les groupes pentadécyle, hexadécyle, heptadécyle, octadécyle, nonadécyle, eicosyle, docosyle et triacontyle.

Le poids moléculaire moyen du polymère de la présente invention est de préférence compris entre 5000 et 30 000, en particulier, entre 6000 et 20 000.

Dans un mode de réalisation préféré, Y est égal à 0 et les groupes R₂ à R₅ représentent des atomes d'hydrogène. De préférence au moins un des radicaux hormis les hydrogènes ont 14 à 32 atomes de carbone.

Dans un autre mode de réalisation, Y n'est pas égal à 0. Les radicaux R₁ à R₉ et R₁₁ à R₁₂ représentent de préférence des atomes d'hydrogène. De préférence, R₁₀ a 14 à 32 atomes de carbone, et le rapport X:Y va de 1:5 à 5:1.

Parmi les produits commerciaux qui peuvent être utilisés dans la présente invention, il est possible de citer en exemple les produits Antaron™ tel que Antaron WP660 dont le monomère CTFA est le tricontanyl-PVP.

La composition de la présente invention comprend le copolymère de vinylpyrrolidone/α-oléfine en une quantité allant de 0,1 à 14 % en poids, de préférence de 0,3 à 3,5 % en poids par rapport au poids total de la composition.

La quantité totale du polymère semi-cristallin et du copolymère de vinylpyrrolidone/α-oléfine doit de préférence être de 1 à 15 % en poids, particulièrement de 2 à 5 % en poids par rapport au poids total de la composition ; en outre, la quantité du polymère cristallisable par rapport à la quantité du copolymère de vinylpyrrolidone/α-oléfine doit de préférence être tel que leur rapport soit de 0,1 à 10, de préférence de 1 à 5.

### C. Phase huileuse

La phase huileuse de la composition selon la présente invention comprend au moins une huile non volatile.

Les huiles non volatiles peuvent être n'importe quelles huiles telles que celles généralement utilisées dans le domaine cosmétique, excepté les huiles volatiles décrites ci-dessous. De préférence, ce sont des huiles ou des polyesters portant au moins deux groupes esters dans la molécule, qui sont liquides à température ambiante (25°C) et à pression atmosphérique (760 mmHg).

Plus préférablement, elles sont choisies parmi les suivantes :
- les composés hydrocarbonés avec une teneur élevée en triglycérides constitués d'acides gras (C₄ à C₂₄) et de glycérine, dont les acides gras peuvent être linéaires ou ramifiés et saturés ou insaturés. Les exemples spécifiques comprennent les triglycérides de l'acide heptanoique, les triglycérides de l'acide octanoique, l'huile de germe de blé, l'huile de maïs, l'huile de tournesol, l'huile de karaté, l'huile de ricin, l'huile d'amande, l'huile de macadamia, l'huile d'abricot, l'huile de soja, l'huile de graines de colza, l'huile de graines de coton, l'huile de luzerne, l'huile de pavot, l'huile de potimarron, l'huile de sésame, l'huile de potiron, l'huile d'avocat, l'huile de noix, l'huile de pépins de raisin, l'huile de graines de cassis, l'huile d'oenothère biennale, l'huile de millet, l'huile d'orge, l'huile de quinoa, l'huile d'olive, l'huile de seigle, l'huile de carthame, l'huile de bancoul, l'huile de passiflore, l'huile de rose musquée, l'huile de graines de coriandre ; ou le tri-(caprylate/caprate) de glycéryle tel que ceux vendus par Cognis sous le nom Myritol 318 ou ceux vendus par Stearineries Dubois sous les noms Triglycerides C8-C10 70/30.
- les esters d'alcools gras tels que le malate de di-isostéaryle, le citrate de tri-isocétyle et le trimellitate de tridécyle.
- les polyesters, par exemple, le dioctanoate de propylène glycol, le diheptanoate de néopentyl glycol et le diisononoate de diéthylène glycol.
- les esters de pentaérythritol, tels que le tétraisostéarate de pentaérythrityle.
- les mélanges de ceux-ci.

En tant qu'huiles non volatiles, il est possible d'utiliser également des absorbants d'UV liquides lipophiles tels que le méthoxycinnamate d'éthylhexyle, le produit commercial Parsol MCX (Roche Vitamins), le produit commercial Escalol 557L (IPS) ou Uvinul MC80 (BASF).

L'huile non volatile de la phase huileuse peut être fournie en une quantité allant de 1 à 99 % en poids, de préférence de 5 à 80 % en poids par rapport au poids total de la phase huileuse.

La présente invention peut en outre contenir des huiles volatiles. Les huiles volatiles qui sont utilisées doivent de préférence avoir un point d'inflammabilité suffisamment élevé.

En tant qu'huiles volatiles, les huiles hydrocarbonées cycliques ou linéaires et/ou les huiles de silicone peuvent être choisies.

Parmi les huiles de silicone, les suivantes peuvent être utilisées, seules ou en mélange.
- Les huiles de silicone cycliques volatiles ayant 3 à 8, de préférence 4 à 6 atomes de silicium, par exemple, le cyclotétradiméthylsiloxane, le cyclopentadiméthylsiloxane ou le cyclohexadiméthylsiloxane, et les cyclocopolymères de type diméthylsiloxane/méthylalkylsiloxane, tel que Silicon FZ 3109 de Union Carbide, qui est un cyclocopolymère de diméthylsiloxane/méthyloctylsiloxane.
- Les silicones volatiles de revêtement ayant 2 à 9 atomes de silicium, par exemple, l'hexaméthyldisiloxane ou le PDMS de faible viscosité (1 cSt). D'autres exemples comprennent les alkyltrisiloxanes tels que le hexylheptaméthyltrisiloxane ou l'octylheptaméthyltrisiloxane.

La phase huileuse est de préférence en une quantité telle que le poids total du polymère semi-cristallin et du copolymère de vinylpyrrolidone/α-oléfine par rapport à la phase huileuse soit tel que leur rapport soit de 0,01 à 1,5, de préférence de 0,02 à 0,6.

La composition de la présente invention peut également comprendre un émulsifiant et une phase aqueuse.

Les émulsifiants utilisés dans la présente invention peuvent être de n'importe quel type pourvu qu'ils aient été utilisés de façon conventionnelle ou qu'ils puissent être utilisés dans les domaines cosmétique et dermatologique. L'homme du métier sera capable de choisir les types et les quantités appropriés d'émulsifiants afin de ne pas changer sensiblement les propriétés favorables de la composition de la présente invention.

En tant qu'émulsifiants qui peuvent être utilisés pour la préparation d'émulsions d'eau dans l'huile, on peut mentionner, par exemple, les esters alkyliques ou les éthers de sorbitane, de glycérol ou de sucre ; les tensioactifs de silicone. En tant qu'exemples de tensioactifs de silicone, on peut citer par exemple, les copolyols de diméthicone tels que le produit vendu sous le nom KF6017 par la société Shin-Etsu, le mélange constitué du 4-isostéarate de polyglycéryle, du copolyol de cétyldiméthicone et du laurate d'hexyle, vendu sous le nom Abil WE 09, par la société Goldschmidt, le mélange de cyclométhicone et de copolyol de diméthicone, vendu sous les noms DC 5225 C et DC 3225 C par la société Dow Corning ou vendu sous le nom Abil EM 97 par la société Goldschmidt, et les copolyols d'alkyldiméthicone tels que le copolyol de laurylméthicone vendu sous le nom "Dow Corning 5200 Formulation Aid" par la société Dow Corning et le copolyol de cétyldiméthicone vendu sous le nom Abil EM 90, par la société Goldschmidt. Il est également possible d'y ajouter un ou plusieurs co-émulsifiants, qui peuvent être choisis avantageusement parmi le groupe comprenant les esters d'un acide gras à chaîne ramifiée et d'un polyol, et particulièrement les esters d'un acide gras à chaîne ramifiée et de glycérol et/ou de sorbitane, et, par exemple, l'isostéarate de polyglycéryle, tel que le produit vendu sous le nom Isolan GI 34 par la société Goldschmidt, l'isostéarate de sorbitane, tel que le produit vendu sous le nom Arlacel 987 par la société ICI, et l'isostéarate de sorbitane et de glycérol, tel que le produit vendu sous le nom Arlacel 986 par la société ICI, et les mélanges de ceux-ci.

Pour les émulsions d'huile dans l'eau, les exemples d'émulsifiants qui peuvent être mentionnés comprennent les émulsifiants non ioniques tels que les esters d'acide gras oxyalkylé (plus particulièrement polyoxyéthylé) de glycérol ; les esters d'acide gras oxyalkylé de sorbitane ; les esters d'acide gras oxyalkylé (oxyéthylé et/ou oxypropylé) ; les éthers d'alkyle gras oxyalkylé (oxyéthylé et/ou oxypropylé) ; les esters de sucre, par exemple, le stéarate de sucrose, et les mélanges de ceux-ci tels que, par exemple, le mélange de stéarate de glycéryle et de stéarate de PEG 100, vendu sous le nom Aracel 165 par la société Uniqema.

Si la composition de la présente invention comprend une phase aqueuse, la phase aqueuse peut comprendre, hormis l'eau, des solvants tels que des alcools primaires contenant 1 à 6 atomes de carbone, tels que l'éthanol, ou des polyols tels que le butylène glycol, la glycérine, le sorbitol, l'hexylène glycol, le propylène glycol ou l'isopropylène glycol, ou des sucres tels que le glucose ou le fructose.

La composition de la présente invention peut en outre contenir des additifs tels que ceux qui sont utilisés conventionnellement dans les domaines cosmétique ou dermatologique, dans une proportion qui n'engendre pas d'impact négatif sur les propriétés avantageuses de la composition selon la présente invention. Les exemples de tels additifs comprennent, par exemple, les matières premières à base d'huile autres que les huiles mentionnées ci-dessus, telles que les cires, les agents gélifiants hormis les polymères mentionnés ci-dessus, en particulier les cires semi-cristallines ayant un point de fusion inférieur à 50°C, les antioxydants, les parfums, les huiles essentielles, les conservateurs, les agents actifs cosmétiques lipophiles ou hydrophiles, les agents mouillants, les agents de remplissage (poudres organiques ou particules minérales), les vitamines, les colorants, les acides gras essentiels, les sphingolipides, les agents autobronzants tels que le DHA, les crèmes solaires et analogues. Ces additifs peuvent être compris dans la composition en une quantité, par exemple, de 0 à 10 % en poids.

Tandis que la composition de la présente invention peut être fabriquée par divers procédés, afin d'empêcher la solidification due à l'agent gélifiant, la structure gélifiée de la phase huileuse doit être détruite ou la progression de la gélification doit être empêchée en agitant pendant la gélification de la phase huileuse pour former une structure sous forme de gel fin.

De préférence, la composition de la présente invention est préparée à l'aide du procédé de fabrication suivant :
- après formation d'une composition solide de la même manière que le procédé de fabrication d'un bâton ou d'un produit de type compact, elle est malaxée par des moyens de cisaillement tels qu'un broyeur à trois cylindres.
- après chauffage et fusion de la composition comprenant un agent gélifiant, des moyens appropriés d'agitation sont utilisés pour refroidir tout en agitant.
- après chauffage et fusion de la composition comprenant un agent gélifiant, elle est passée dans une extrudeuse.

Par exemple, le procédé décrit dans EP 0 755 668 et EP 0 745 376 peut être utilisé.

De plus, d'autres moyens qui sont publiquement connus dans le domaine peuvent être utilisés pour former une structure sous forme de gel fin. Par exemple, pendant la gélification, des moyens constituant à maintenir une température inférieure à la température à laquelle elle fond complètement, à mettre dans un état dissous à l'air libre, à causer un mouvement fin tel que des vibrations ultrasoniques, ou à effectuer une cavitation peuvent être utilisés seuls ou combinés.

La composition de la présente invention peut être utilisée en tant que composition cosmétique ou agent externe destinée à une application sur la peau, et est particulièrement adaptée pour être une composition cosmétique destinée à la protection, au soin, au maquillage ou au démaquillage et/ou au nettoyage de la peau (y compris le cuir chevelu), des cheveux, des ongles et/ou des muqueuses. En particulier, la composition peut constituer un démaquillant ou un fond de teint.

### Exemples

Ci-après, la présente invention est décrite en détail. Sauf indication contraire, "%" fait référence à un % en poids.

### I) Exemples de formulation de composition

Dans les exemples suivants 3 à 5, la gélification des divers types de phases huileuses a été essayée selon les procédés suivants, et la destruction des structures gélifiées a été essayée, pour préparer les compositions :
1. Chauffer les huiles à 75°C tout en agitant à l'aide d'une palette.
2. Ajouter le polymère et agiter pendant 20 minutes à 75°C.
3. Stocker le produit brut dans une étuve à 25°C pendant 12 heures.
4. Passer une fois dans un broyeur à trois cylindres.

Les propriétés et les viscosités des compositions résultantes ont été mesurées à diverses températures.

### Exemple 1 : Gélification d'huiles volatiles (exemple de référence)

| | A1 | A2 | A3 | A4 | A5 | B1 | B2 | B3 | B4 | B5 |
|---|---|---|---|---|---|---|---|---|---|---|
| Polymère semi-cristallin ¹ (Point de fusion : 49°C) | 6 % | 3 % | - | - | - | 6 % | 3 % | - | - | - |
| Copolymère de polyvinylpyrrolidone /α-oléfine ² (Point de fusion : 63°C) | - | 3 % | 6 % | 3 % | - | - | 3 % | 6 % | 3 % | - |
| Polymère semi-cristallin ³ (Point de fusion : 66°C) | - | - | - | 3 % | 6 % | - | - | - | 3 % | 6 % |
| Cyclopentadiméthylsiloxane ⁴ | 94 % | 94 % | 94 % | 94 % | 94 % | - | - | - | - | - |
| Isododécane ⁵ | - | - | - | - | - | 94 % | 94 % | 94 % | 94 % | 94 % |
| Aspect | Non gélifié | | | | | | | | | |

| | | | | | | | | | | |
|---|---|---|---|---|---|---|---|---|---|---|
| ¹ Homopolymère d'acrylate de stéaryle (Intelimer IPA-13.1 de Landec) | | | | | | | | | | |
| ² Antaron WP-660 | | | | | | | | | | |
| ³ Homopolymère d'acrylate de béhényle (Intelimer IPA-13.6 de Landec) | | | | | | | | | | |
| ⁴ Fluide DC 245 (Dow-Corning) | | | | | | | | | | |
| ⁵ Permethyl 99A (Permethyl) | | | | | | | | | | |

### Exemple 2 : Gélification d'huile de noyaux d'abricot (présente invention)

| | C1 Produit comp. | C2 Produit comp. | C3 Produit comp. | C4 Présente invention | C5 Produit comp. |
|---|---|---|---|---|---|
| Polymère semi-cristallin ¹ (Point de fusion : 49°C) | 6 % | 3 % | - | - | - |
| Copolymère de polyvinylpyrrolidone /α-oléfine ² (Point de fusion : 63°C) | - | 3 % | 6 % | 3 % | - |
| Polymère semi-cristallin ³ (Point de fusion : 66°C) | - | - | - | 3 % | 6 % |
| Huile de noyaux d'abricot⁶ | 94 % | 94 % | 94 % | 94 % | 94 % |
| Aspect à 25°C (après 24 h) | Gel | Gel | Gel | Gel | Gel |
| Aspect à 45°C (après 24 h) | Liquide | Liquide | Liquide | Gel | Gel léger |
| Viscosité à 25°C (après 24 h) * | 44,7 sur M3 1712 cps | 51,7 sur M2 349 cps | 34,7 sur M2 216 cps | 26,2 sur M3 915 cps | 19,7 sur M3 661 cps |
| Viscosité à 45°C (après 24 h) * | Trop fluide, non mesurée | | | 13,5 sur M3 | 8,87 sur M3 |
| | | | | 450 cps | 283 cps |

| | | | | | |
|---|---|---|---|---|---|
| * Les résultats de la viscosité sont les viscosités mesurées aux températures respectives après avoir laisser aux différentes températures pendant 24 heures. La partie du haut donne les lectures sur le viscosimètre RM180 et la partie du bas donne les valeurs après conversion des unités en centipoises. | | | | | |
| ⁶ Huile brute d'amandes d'abricot MP301 (Safic-Alcan) | | | | | |

Ce tableau montre que seulement un polymère ayant une température de fusion d'au moins 50°C permet d'obtenir la composition revendiquée.

### Exemple 3 : Gélification du triglycéride d'acide caprylique et caprique (présente invention)

| | D1 Produit comp. | D2 Produit comp. | D3 Produit comp. | D4 Présente invention | D5 Produit comp. |
|---|---|---|---|---|---|
| Polymère semi-cristallin ¹ (Point de fusion : 49°C) | 6 % | 3 % | - | - | - |
| Copolymère de polyvinylpyrrolidone /α-oléfine ² (Point de fusion : 63°C) | - | 3 % | 6 % | 3 % | - |
| Polymère semi-cristallin ³ | - | - | - | 3 % | 6 % |
| (Point de fusion : 66°C) | | | | | |
| Triglycéride d'acide caprylique/caprique ⁷ | 94 % | 94 % | 94 % | 94 % | 94 % |
| Aspect à 25°C (après 24 h) | Gel | Gel | Gel | Gel | Gel |
| Aspect à 45°C (après 24 h) | Liquide | Liquide | Liquide | Gel | Gel léger |
| Viscosité à 25°C (après 24 h) * | 41,4 sur M3 1542 cps | 15,0 sur M3 483 cps | 35,2 sur M2 216 cps | 29,6 sur M3 1076 cps | 22,2 sur M3 746 cps |
| Viscosité à 45°C (après 24 h) * | Trop fluide, non mesurée | | | 14,1 sur M3 450 cps | 12,8 sur M3 417 cps |

| | | | | | |
|---|---|---|---|---|---|
| ⁷ Myritol 318 (Cognis) | | | | | |

L'exemple 1 (exemple de référence) montre que les huiles volatiles ne se gélifient pas même lorsqu'elles sont utilisées conjointement avec un polymère semi-cristallin et un copolymère de polyvinylpyrrolidone/α-oléfine. De plus, dans les exemples 2 ou 3, quelle que soit l'huile non volatile utilisée, si l'un du polymère semi-cristallin et du copolymère de polyvinylpyrrolidone/α-oléfine est enlevé, la stabilité du gel est perdue aux températures élevées, le produit devient liquide (compositions C1 à C3 et D1 à D3), ou n'a pas une viscosité suffisante (compositions C5 et D5), mais lorsque l'on utilise à la fois le polymère semi-cristallin et le copolymère de polyvinylpyrrolidone/α-oléfine (compositions C4 et D4), une structure gélifiée stable est obtenue même à des températures élevées.

### III) Exemple de formulation de composition cosmétique

### Exemple 4 : Huile nettoyante sous forme de gel

### Procédé de préparation :

1. La phase huileuse, le tensioactif et la phase aqueuse ont été chauffés à 75°C tout en agitant à l'aide d'une palette.
2. Les polymères autres que la silice diméthylsilylate ont été ajoutés et complètement dissous.
3. La silice diméthylsilylate a été dispersée à l'aide d'une palette et complètement imbibée dans le produit brut.
4. La palette a été changée par un Rayneri, et le produit brut a été dispersé jusqu'à ce qu'il soit lisse à 75°C.
5. Le produit brut a été laissé reposer à température ambiante.
6. Après 24 heures, le produit résultant a été passé dans un broyeur à trois cylindres.

| | E1 Prod. comp. | E2 Présente Invention | E3 Prod. comp. | E4 Prod. comp. | E5 Prod. comp. | E6 Produit comp. |
|---|---|---|---|---|---|---|
| Phase huileuse | | | | | | |
| - Triglycéride d'acide caprylique/caprique | 38,5 % | 38,5 % | 39,5 % | 38,5 % | 38,5 % | 41,5 % |
| - Isohexadécane | 15 % | 15 % | 15 % | 15 % | 15 % | 15 % |
| - Huile de graines de jojoba ⁸ | 10,5 % | 10,5 % | 10,5 % | 10,5 % | 10,5 % | 10,5 % |
| - Huile de noyaux d'abricot | 10,5 % | 10,5 % | 10,5 % | 10,5 % | 10,5 % | 10,5 % |

| Tensioactif | | | | | | |
|---|---|---|---|---|---|---|
| Triisostéarate de glycéryle POE-20 ⁹ | 16 % | 16 % | 16 % | 16 % | 16 % | 16 % |

| Phase aqueuse | | | | | | |
|---|---|---|---|---|---|---|
| Eau | 0,5 % | 0,5 % | 0,5 % | 0,5 % | 0,5 % | 0,5 % |
| Polymère | | | | | | |
| - Silice Diméthylsilylate ¹⁰ | 6 % | 6 % | 6 % | 6 % | 6 % | 6 % |
| - Polymère semi-cristallin ¹ (Point de fusion : 49°C) | 2 % | - | - | - | - | - |
| - Polymère semi-cristallin ³ (Point de fusion : 66°C) | - | | | | | |
| - Tricontanyl PVP¹¹ | 1 % | 1 % | 2 % | 2 % | 2 % | 2 % |
| - Copolymère Vp/hexadécène ¹² | - | - | - | - | - | - |
| - Copolymère Vp/eicosène ¹³ | - | - | - | - | 1 % | - |
| Aspect 24 h après préparation | Crème opalescente jaune pâle | | | | | Fluide transparent jaune pâle |
| Viscosité cylindre de lecture centipoise | M3 : 28,0 983 cps | M3 : 39,7 1500 cps | M2 : 38,8 248 cps | M2 : 49,3 324 cps | M2 : 55,5 382 cps | M2 : 35,6 221 cps |
| Centrifugation (1 h, g = 900) | Sépar. | Sépar. | Sépar. | Sépar. | Sépar. | Sépar. |
| Stabilité 2 mois après préparation | Instable | Stable | Stable | Instable | Instable | Instable |

| | | | | | | |
|---|---|---|---|---|---|---|
| ⁸ Huile de jojoba pure dorée (Desert Whale) | | | | | | |
| ⁹ Emalex GWIS-320 EX (Nihon Emulsion) | | | | | | |
| ¹⁰ Aerosil R 972 (Degussa) | | | | | | |
| ¹¹ Antaron WP-660 ; point de fusion 63°C ; aspect : flocons solides | | | | | | |
| ¹² Antaron V-216 ; aspect : fluide visqueux | | | | | | |
| ¹³ Antaron V-220 ; point de fusion 35°C ; aspect : flocons cireux | | | | | | |

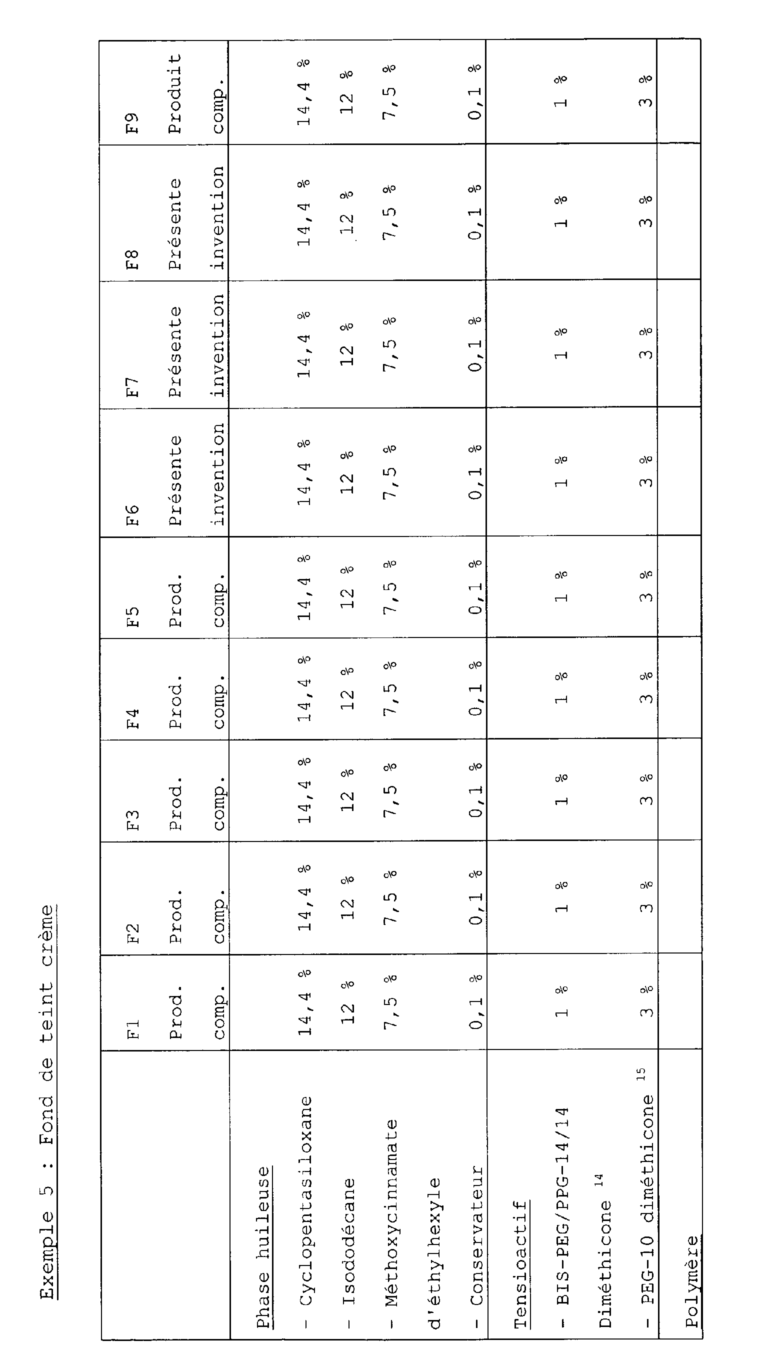

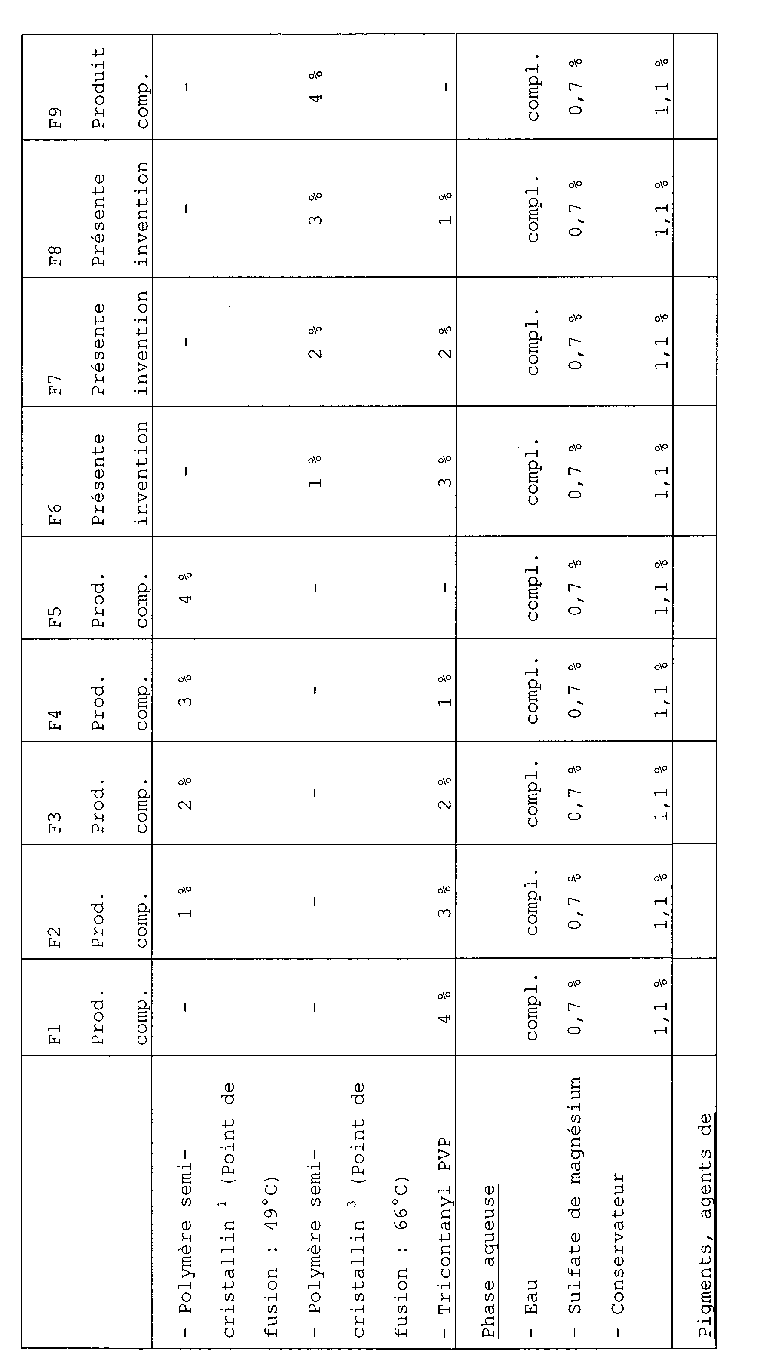

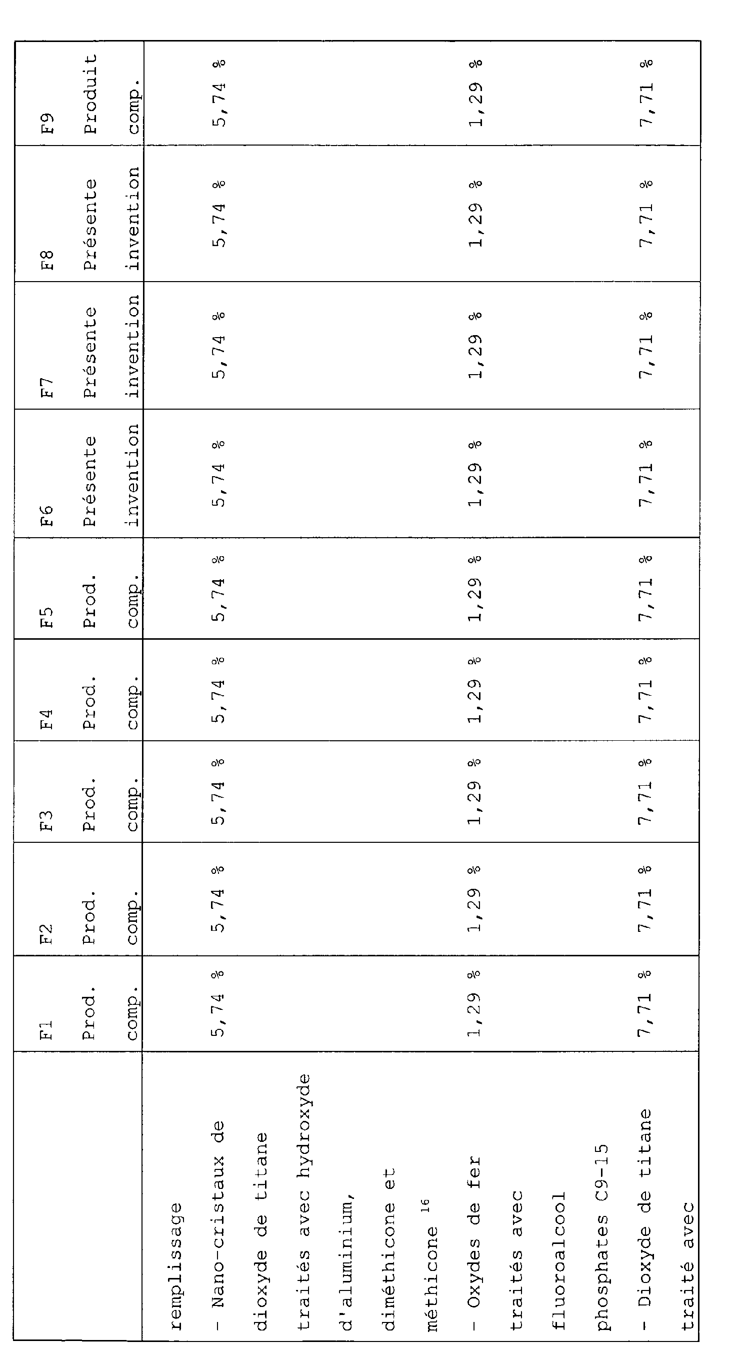

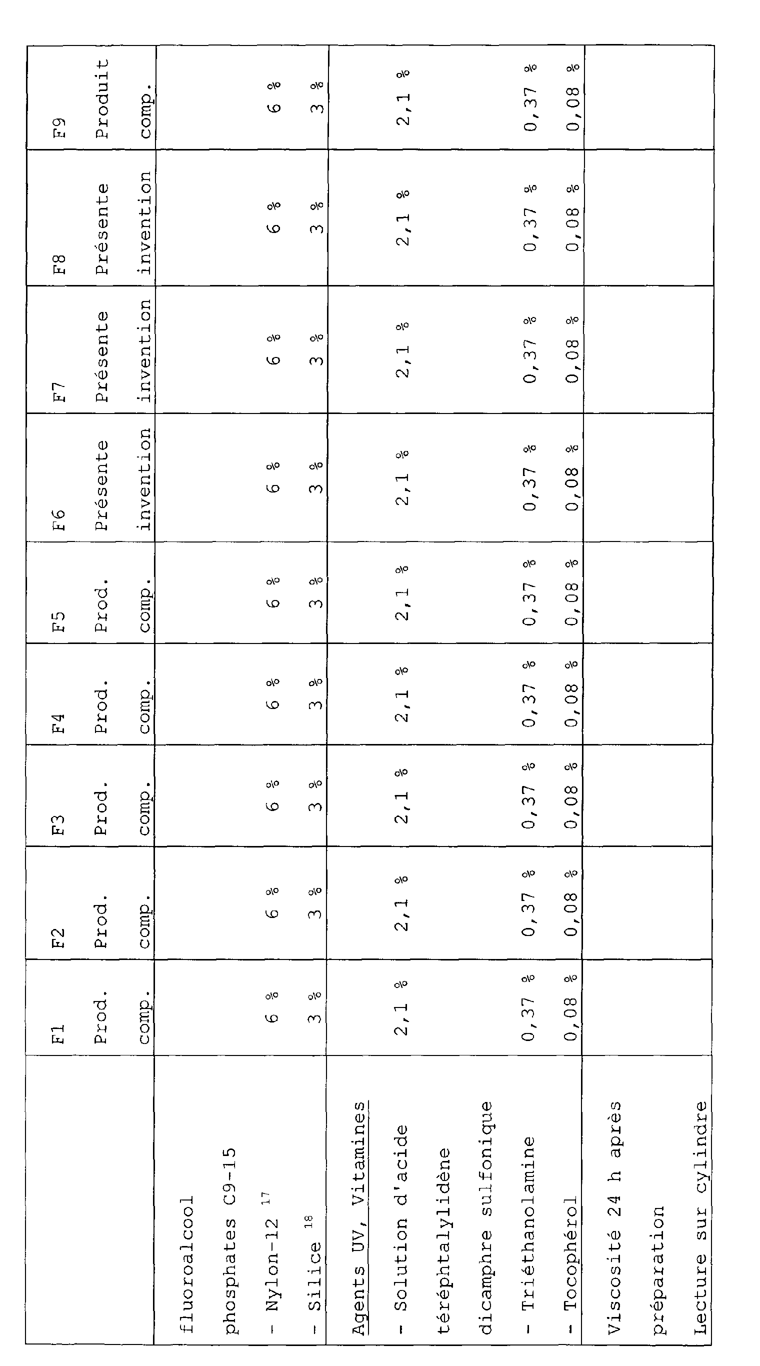

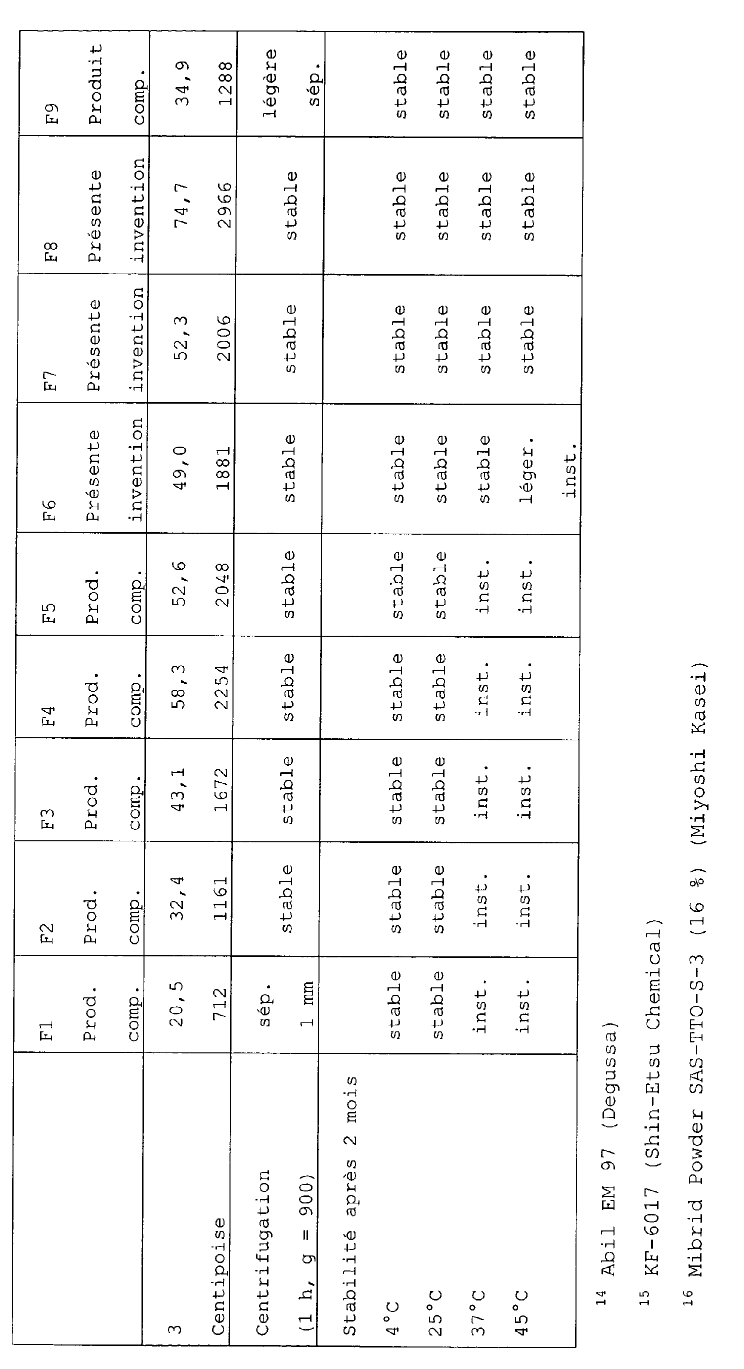

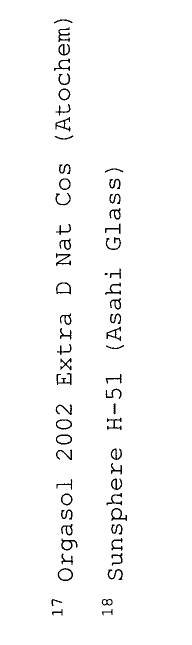

### Procédé :

| Phase A1 | |
|---|---|
| BIS-PEG/PPG-14/14 Diméthicone | 1 % |
| PEG-10 Diméthicone | 3 % |
| Cyclopentasiloxane | 8,4 % |
| Isododécane | 12 % |
| Méthoxycinnamate d'éthylhexyle | 7,5 % |
| Conservateur | 0,1 % |

| Phase A2 | |
|---|---|
| Polymère semi-cristallin (Intelimer | 3 % |
| IPA-13.6), point de fusion 66°C | |
| Tricontanyl PVP | 1 % |

| Phase A3 | |
|---|---|
| Nano-cristaux de dioxyde de titane | 5,74 % |
| traités avec de l'hydroxyde | |
| d'aluminium, de la diméthicone et de la méthicone | |

| Phase A4 | |
|---|---|
| Oxydes de fer traités avec des | 1,29 % |
| fluoroalcool phosphates C9-15 | |
| Dioxyde de titane traité avec des | 7,71 % |
| fluoroalcool phosphates en C9-15 | |
| Cyclopentasiloxane | 6 % |

| Phase A5 | |
|---|---|
| Nylon-12 | 6 % |
| Silice | 3 % |

| Phase A6 | |
|---|---|
| Tocophérol | 0,08 % |

| Phase B | |
|---|---|
| Eau | complément à |
| | 100 % |
| Sulfate de magnésium | 0,7 % |
| Conservateur | 1,1 % |

| Phase C | |
|---|---|
| Solution d'acide téréphtalylidène | 2,1 % |
| dicamphre sulfonique | |

### Préparation :

Phase A4 : Le pigment et le cyclopentasiloxane ont été passés trois fois dans un broyeur à trois cylindres.
Phase B : B a été complètement dissous et ajusté à 50°C.

### Production :

1. La phase A1 a été versée dans un bécher principal, et complètement dissoute à température ambiante à l'aide d'un agitateur magnétique.
2. La phase A2 a été ajoutée et dissoute à une température comprise entre 60 et 70°C.
3. La phase A3 a été ajoutée et dispersée à l'aide d'un homogénéisateur Mizuho (10 minutes).
4. La phase A4 a été ajoutée et dispersée (20 à 30 minutes).
5. La phase A5 a été ajoutée et dispersée (5 minutes).
6. La phase B a été ajoutée et émulsionnée à 50°C (5 minutes).
7. Refroidissement à température ambiante, et les phases A6 et C ont été ajoutées.
8. Transfert dans un mélangeur à palette, et agitation pendant 10 à 20 minutes pour éliminer les bulles.

## Revendications

1. Composition destinée à une application externe, comprenant au moins un polymère semi-cristallin ayant un point de fusion d'au moins 50°C, au moins un copolymère de polyvinylpyrrolidone/α-oléfine ayant un point de fusion d'au moins 50°C, et une phase huileuse contenant au moins une huile non volatile, et prenant la forme d'une pâte ou d'une crème à température ambiante.

2. Composition selon la revendication 1, dont la viscosité à température ambiante est comprise entre 330 centipoises et 35 000 centipoises.

3. Composition selon la revendication 1, prenant la forme d'une pâte ou d'une crème, cette forme étant due au fait que la structure gélifiée a été détruite après gélification de la phase huileuse, ou que la progression de la gélification a été empêchée pendant la gélification de la phase huileuse.

4. Composition selon l'une quelconque des revendications 1 à 3, dans laquelle le polymère semi-cristallin a une structure organique avec une chaîne latérale cristalline et/ou un bloc formé d'une chaîne principale polymérique cristalline, et a un poids moléculaire moyen en nombre d'au moins 2000.

5. Composition selon l'une quelconque des revendications 1 à 4, dans laquelle le polymère semi-cristallin est choisi parmi les homopolymères et les copolymères formés par polymérisation d'au moins un monomère contenant une chaîne cristalline, représentés par la formule (I) : 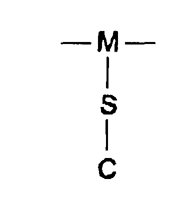 (dans laquelle M représente un atome de la chaîne principale polymérique, S représente un espaceur, et C représente un groupe cristallisable), et la chaîne cristallisable "-S-C" représente une chaîne alkyle comprenant au moins 11 atomes de carbone qui peuvent être fluorés ou perfluorés.

6. Composition selon l'une quelconque des revendications 1 à 5, dans laquelle le polymère semi-cristallin a un point de fusion compris entre 50 et 120°C, et un poids moléculaire moyen en nombre de 2000 à 800 000.

7. Composition selon l'une quelconque des revendications 1 à 6, dans laquelle le polymère semi-cristallin est l'homopolymère de l'acrylate de béhényle.

8. Composition selon l'une quelconque des revendications 1 à 7, dans laquelle le polymère semi-cristallin est présent dans la composition en une quantité allant de 0,1 à 14 % en poids par rapport au poids total de la composition.

9. Composition selon l'une quelconque des revendications 1 à 8, dans laquelle le copolymère de polyvinylpyrrolidone/α-oléfine a un point de fusion compris entre 50 et 70°C.

10. Composition selon l'une quelconque des revendications 1 à 9, dans laquelle le copolymère de polyvinylpyrrolidone/α-oléfine est un polymère représenté par la formule suivante :

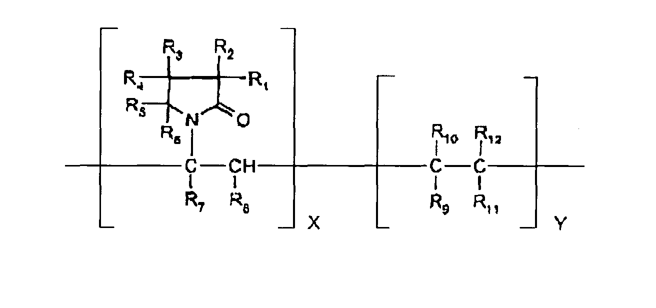

11. Composition selon l'une quelconque des revendications 1 à 10, dans laquelle le copolymère de polyvinylpyrrolidone/α-oléfine est présent dans la composition en une quantité allant de 0,1 à 14 % en poids par rapport au poids total de la composition.

12. Composition selon l'une quelconque des revendications 1 à 11, dans laquelle l'huile non volatile est présente dans la composition en une quantité allant de 1 à 99 % en poids par rapport au poids total de la composition.

13. Composition selon l'une quelconque des revendications 1 à 12, qui est une émulsion d'eau dans l'huile ou qui est non aqueuse.

14. Composition selon l'une quelconque des revendications 1 à 13, qui est une composition cosmétique.

15. Procédé de préparation d'une composition destinée à une application externe, qui consiste à mélanger au moins un polymère semi-cristallin ayant un point de fusion d'au moins 50°C, et au moins un copolymère de polyvinylpyrrolidone/α-oléfine ayant un point de fusion d'au moins 50°C, avec une phase huileuse comprenant au moins une huile non volatile ; à gélifier ladite phase huileuse ; et à appliquer une force de cisaillement après la gélification ou pendant la gélification pour obtenir une structure gélifiée fragmentée.
